# EUROPÄISCHE PATENTANMELDUNG

(11) **EP 1 202 046 A1**
(43) Veröffentlichungstag der Anmeldung: **02.05.2002**
(21) Anmeldenummer: 00810987.8
(22) Anmeldetag: 25.10.2000
(51) Int. Cl.: G01N 27/00, C12Q 1/68, G01N 33/50

(54) **Verfahren und Sensor zur Bestimmung biologischer Spezies**

(71) Anmelder: ABB RESEARCH LTD., 8050 Zürich (CH)
(72) Erfinder: Strümpler, Ralf, Dr., 5412 Gebenstorf (CH); Rancsò, Christoph, Dr., 12205 Berlin (DE)
(74) Vertreter: ABB Patent Attorneys

(57) **Zusammenfassung**

Bei dem Verfahren werden die zu bestimmenden Spezies (5) mit einer für sie spezifischen Antikörperschicht (4) in Kontakt gebracht. Die Antikörperschicht (4) ist auf einem Resonatorkörper (2) vorgesehen. Infolge der Massenveränderung durch gebundene Spezies (5) erfährt der Resonatorkörper (2) eine Verschiebung seiner Resonanzfrequenz. Mit dem Verfahren kann die Herstellung keimfreier Produkte on-line überwacht werden.

## Beschreibung

Die Erfindung betrifft ein Verfahren und einen Sensor zur selektiven quantitativen Bestimmung biologischer Spezies.

Bei der Herstellung keimfreier Produkte, beispielsweise in der Nahrungsmittelindustrie, wird bis heute die Abwesenheit von toxischen Spezies, z.B. Bakterien, Pilzen etc. durch Untersuchung von Stichproben nachgewiesen. Chemische bzw. immunologische Untersuchungsmethoden sind nicht schnell genug, um eine kontinuierliche Überwachung einer Produktionsstrasse zuzulassen.

Dill, K. et al. in J. Biochem Biophys Methods 1999, 41, S. 61-67 beschreiben ein Verfahren zur Bestimmung von Salmonellen auf Geflügelfleisch. Das Verfahren beruht auf dem Binden von Biotin und fluorescein-markierten Antikörpern an Salmonellen. Der entstehende Immunkomplex wird durch eine Filtration über eine mit Biotin beschichtete Nitrozellulosemembran isoliert und erfährt eine weitere Bindungsreaktion von Anti-Fluorescein-Urease an den Komplex. In der Gegenwart von Harnsäure wandelt die Urease das Substrat in Ammoniak und CO₂ um, was zu einer Änderung des pH-Werts führt. Diese Änderung kann mit einem Silizium-Chip in einer potentiometrischen Sensoranordnung gemessen werden. Es ist offensichtlich, dass diese komplizierte Methode sich nicht für eine kontinuierliche Überwachung eignet.

Seo, K.H. et al. in J. Food Prot. 1999, 62 (5),S. 431-437 beschreiben ein anderes Verfahren zur Salmonellenbestimmung mit Hilfe eines Interferometers. Die beiden Kanäle eines planaren Wellenleiters sind mit Antikörpern beschichtet, eine Seite mit Salmonellenantikörpern und die andere Seite mit Humanimmunoglobulin G als Referenz. Wenn Salmonellen aus einer Probe an die Antikörper binden, ergibt sich eine Phasenverschiebung zwischen den beiden Kanälen, die ausgewertet wird. Auch dieses Verfahren eignet sich wegen der Dauer jeder Einzelmessung nicht für kontinuierliche Überwachungen in der Produktion.

Heim, S. et al. in Biosens. Bioelektron. 1999, 14 (2), S. 187-193 beschreiben ein mikrobiologisches Sensorsystem, das auf der Messung des Sauerstoffverbrauchs während des mikrobiellen Stoffwechsels beruht. Dabei handelt es sich naturgemäss um ein langsames Verfahren.

Mikrosensoren zur Online-Bestimmung chemischer Substanzen sind bekannt. Baltes, H. et al. in Proc. 29^{th} Europ. Solid-State Device Conference 1999, S. 29-37 beschreiben verschiedene Sensortypen für eine elektronische Nase, d.h. ein Gerät zur Bestimmung von flüchtigen chemischen Substanzen in der Luft. Dickert, F.L. et al. in Adv. Mater. 1993, 5, Nr. 12, S. 887-895 beschreiben Mikrosensoren zur Bestimmung von Lösungsmitteldämpfen etc.

Fritz J. et al. in Science Vol. 288 vom 14.4.2000 beschreiben einen Sensor zum Detektieren von Oligonukleotiden, die mittels spezifischer Antikörper auf Silizium-Federzungen immobilisiert werden und diese durch elektrostatische, sterische oder hydrophobe Abstossung biegen. Die resultierende Biegung der Federzungen wird optisch mit einem reflektierten Laserstrahl gemessen.

Für die Online-Überwachung der Produktion keimfreier Produkte besteht aber nach wie vor die Aufgabe, ein Verfahren und einen Sensor zu finden, welche den Anforderungen an Selektivität, Messgenauigkeit und Schnelligkeit genügen.

Erfindungsgemäss wird dies dadurch erreicht, dass ein Resonanzkörper, der mit einer Schicht mit Antikörpern für die zu bestimmende Spezies versehen ist, zu Schwingungen angeregt und seine Resonanzfrequenz gemessen wird. Ein Sensor zum Durchführen dieses Verfahrens besitzt einen Resonanzkörper und eine auf dessen Oberfläche angeordnete Schicht mit Antikörpern für die zu bestimmende Spezies, sowie Mittel zur Schwingungsanregung und zur Messung der Resonanzfrequenz des Resonanzkörpers.

Im folgenden werden anhand der beiliegenden Zeichnungen bevorzugte Ausführungsbeispiele der Erfindung beschrieben.
Es zeigen
- Fig. 1: eine schematische perspektivische Darstellung eines Federzungensensors,
- Fig. 2: eine schematische Schnittdarstellung des in Fig. 1 gezeigten Sensors,
- Fig. 3: eine Draufsicht auf den Sensor der Fig. 1,
- Fig. 4: eine schematische perspektivische Darstellung des Resonanzkörpers eines piezoelektrischen Sensors,
- Fig. 5: eine entsprechende Darstellung des in Fig. 4 gezeigten Sensors mit Antikörpern und an diese gebundenen Bakterien.

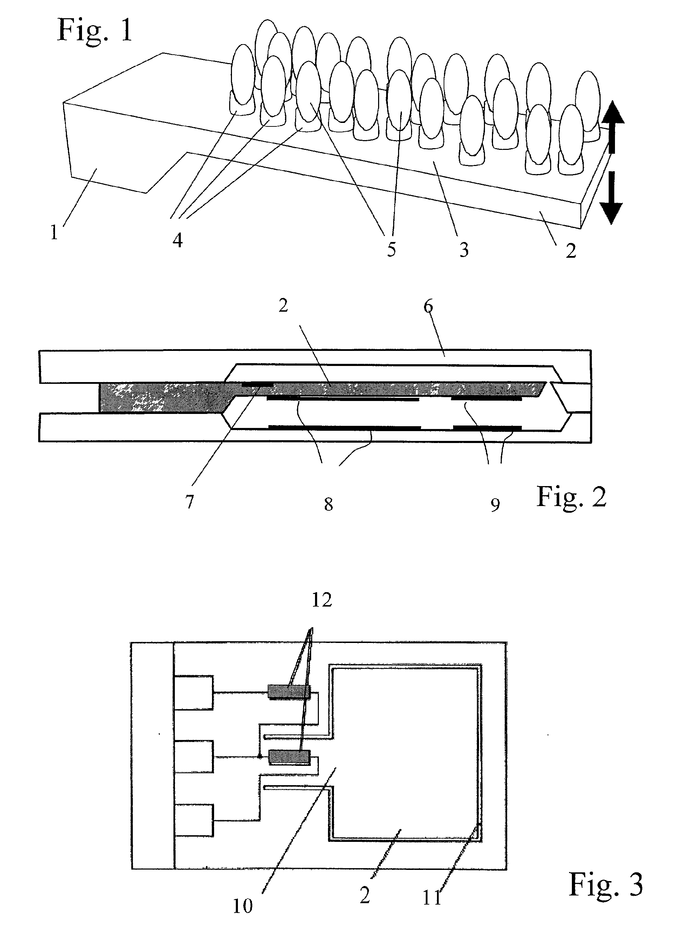

Eine erste bevorzugte Form des erfindungsgemässen Sensors ist der in den Fig. 1-3 gezeigte Federzungensensor. Bei diesem Sensor besteht der Resonanzkörper im wesentlichen aus einer Basis 1 und einer sich von dieser freitragend erstreckenden elastischen Federzunge 2. Basis und Federzunge bestehen aus einem Stück eines federelastischen Materials, wie beispielsweise aus Silikon, Keramik, Plastik, Metall oder Glas, vorzugsweise versehen mit einer aufgesputterten oder aufgalvanisierten Beschichtung. Die Federzunge kann mit unterschiedlichen Mitteln zu Schwingungen angeregt werden, die im folgenden näher beschrieben werden.

Auf der oberen Oberfläche 3 der Federzunge 2 sind Antikörper 4 immobilisiert. Die Antikörper 4 sind spezifisch für die zu bestimmenden Bakterien 5 oder dergleichen. Kommen die zu bestimmenden Bakterien mit der Antikörperschicht 4 in Kontakt, binden die Bakterien an die Antikörper. Dadurch verändert sich die Masse der Federzunge und als Folge auch ihre Resonanzfrequenz.

Wie in Fig. 2 ersichtlich ist die Basis 1 des Resonanzkörpers so in einem Gehäuse 6 eingespannt, dass die Federzunge 2 in der durch die Pfeile in Fig. 1 angegebenen Richtung frei schwingen kann. In Fig. 3 ist die Form der Federzunge ersichtlich, die durch einen Steg 10 mit der Basis 1 verbunden ist. Die Federzunge 2 ist durch einen Luftspalt 11 von umgebenden Material getrennt.

Die Abmessungen der Federzunge 2 betragen typischerweise ca. 150 x 20 x 2 µm ( Länge x Breite x Dicke ). Seine Resonanzfrequenz liegt typischerweise im Bereich zwischen 100 kHz und 1 MHz.

Eine Federzunge mit diesen Abmessungen und einer Resonanzfrequenz von 400 kHz besitzt eine Empfindlichkeit von ca. 5 · 10⁻¹² g, was einer Verschiebung der Resonanzfrequenz von 1 Hz entspricht. Ein Bakterium von 2-3 µm Länge und 1 µm Durchmesser wiegt etwa 2 · 10⁻¹² g. Folglich können mit einer solchen Federzunge etwa drei bis fünf einzelne Bakterien gemessen werden.

Gemäss einer ersten Ausführungsform des Sensors wird die Federzunge elektrothermisch zu Schwingungen angeregt. Zu diesem Zweck ist, wie in Fig. 2 gezeigt, in der Nähe des Ansatzes der Federzunge ein Widerstand 7, z.B. ein Metallfilm oder hoch dotiertes Silizium aufgebracht. Beim Heizen dieses Widerstand wird die Federzunge wegen unterschiedlicher Ausdehnung von Widerstand und Federzunge verbogen (bimorpher Effekt). Wegen der Kleinheit der Strukturen erfolgt die Aufheizung und damit die Verbiegung sehr schnell, so dass man die Federzunge zu Schwingungen angeregt wird.

Alternativ kann die Federzunge elektrostatisch angeregt werden. Zu diesem Zweck sind, wie ebenfalls in Fig. 2 gezeigt, auf der Unterseite der Federzunge und auf dem Gehäuse darunter Elektroden 8 angebracht. Bei Anlegen einer Spannung wird durch elektrostatische Kräfte die Federzunge angezogen.

Folgende weitere Anregungsmöglichkeiten kommen in Betracht: Piezoelektrische Anregung mittels einer auf der Federzunge aufgebrachten dünnen Schicht aus einem piezoelektrischen Material, z.B. ZnO oder PZT (PbZrTiO₃). Bei Anlegen einer Spannung dehnt sich dieses Material aus, bzw. zieht sich zusammen. Dadurch wird die Federzunge in Schwingung versetzt. Für eine elektromagnetische Anregung ist die Federzunge oder eine darauf aufgebracht Schicht aus ferromagnetische Material hergestellt. Eine in dem Substrat eingelassene Spule (spiralförmig oder Mäander) zieht bei Stromfluss die Federzunge an.

Die Messung der die Schwingungsfrequenz der Federzunge erfolgt bei einer Ausführungsform des Sensors kapazitiv. Auf der Unterseite der Federzunge und auf dem unterliegenden Substrat sind, ähnlich wie für die elektrostatische Anregung Elektroden 9 angebracht, deren Kapazität in an sich bekannter Weise gemessen wird. Im Falle der elektrostatischen Anregung können die Anregungselektroden 8 gleichzeitig zur Bestimmung der Schwingungsfrequenz benutzt werden.

Alternativ kann die Schwingungsfrequenz piezoresistiv gemessen werden. In die Federzunge und das feststehende Substrat sind, wie in Fig. 3 gezeigt, piezoresistive Widerstände 12 eingebracht die als Wheatstone-Brücke verschaltet sind. In Silizium können solche Widerstände einfach durch z.B. p-Dotierung erreicht werden, da Silizium selbst piezoresistiv ist. Einer der Widerstände befindet sich in dem Teil der Federzunge, der bei Anregung stark gebogen wird. Die auftretenden Druck- und Zugspannungen im Material bewirken Widerstandsänderungen, die mit der Brückenschaltung sehr empfindlich gemessen werden können.

Eine weitere Alternative ist die induktive Messung der Schwingungsfrequenz. Die Federzunge oder eine darauf aufgebracht Schicht sind aus ferromagnetischem Material hergestellt. Eine in dem Substrat eingelassene Spule erfährt eine Impedanzänderung, während sich die Federzunge bewegt.

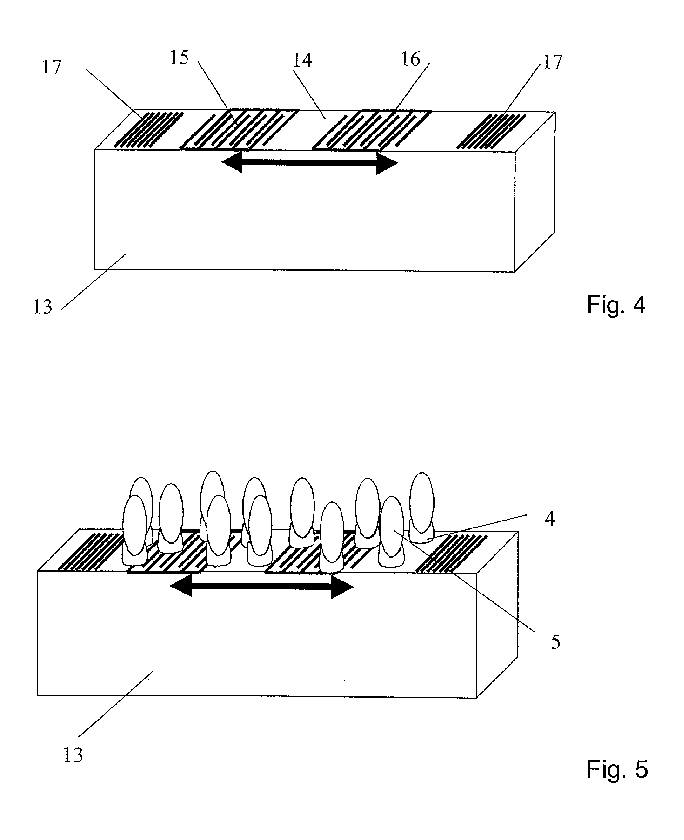

Eine andere Form des erfindungsgemässen Sensors ist der in Fig. 4 und 5 gezeigte piezoelektrische Sensor. Ein piezoelektrisches Substrat 13, wie beispielsweise Quarz, Zinkoxyd etc., besitzt an einer Oberfläche 14 aufgebrachte Anregungselektroden 15 und Detektorelektroden 16. Zu beiden Seiten der beiden Elektroden sind Reflektoren 17 angebracht.

Die Elektroden sind kammförmig ausgebildet. Die Anregungselektrode erzeugt mit einem angelegten Signal eine akustische Oberflächenwelle in Richtung der eingezeichneten Pfeile, die durch die Detektorelektrode erfasst wird. Die durch die Reflektoren 10 begrenzte Strecke für die Schwingungen führt zur Ausbildung einer stehenden Welle.

Wie in der ersten Ausführungsform sind auch beim piezoelektrischen Sensor der Fig. 2 auf der Oberfläche, auf der die akustischen Wellen erzeugt und gemessen werden, Antikörper 4 gegen die zu bestimmende Spezies fixiert. An diese lagern beispielsweise Bakterien 5 etc. an, was hier ebenfalls durch eine Veränderung der Masse zu einer Verschiebung der Resonanzfrequenz führt.

Die Antikörper können sehr fest auf verschiedenen Substraten fixiert werden. Eine einfache Möglichkeit besteht darin, eine Kunststoffoberfläche mit einer Antikörperlösung zu benetzen und anschliessend zu trocknen. Die Bindung der Antikörper an die Kunststoffoberfläche erfolgt durch Seitenketten derart, dass genügend Antigen-Bindungsmöglichkeiten an den freien Oberflächen der Antikörper vorhanden sind.

Die Antikörper können auch an Mikrokügelchen aus Agarose oder Silikaten mittels Protein A gebunden werden. Das Protein A bindet an den konstanten Teil des Antikörpermoleküls in nicht kovalenter Form. Diese Bindung verwandelt sich danach in eine kovalente Bindung als Folge einer zusätzlichen chemischen Reaktion.

Beispiele für Bakterien, die mit der erfindungsgemässen Methode bestimmt wurden, sind

| | |
|---|---|
| Enterobakterien (stäbchenförmig) | 2-3 µm |
| Salmonellen (stäbchenförmig) | 2-3 µm |
| Skigella ( stäbchenförmig) | 2-3 µm |
| Staphylokokken | 0,8-1,2 µm |
| Legionella pneumophila | |

Bestimmte Bakterien oder Pilze erzeugen toxische Stoffwechselprodukte, die ebenfalls mittels Antikörpern spezifisch bestimmt wurden. So erzeugt z.B. Clostridium perfringens Typ A ein Enterotoxin, ein hitzeempfindliches Protein mit einem Molekulargewicht von 35000, zu dem es Antikörper gibt. Fusarium erzeugt Deoxynivalmol (DON) ein im Weizen auftretendes Gift, das ebenfalls mit Antikörpern immobilisiert werden kann.

Ausser den genannten Mikroorganismen und deren toxischen Stoffwechselprodukten wurden auch Blütenpollen mit den erfindungsgemässen Sensoren erfolgreich gemessen.

## Patentansprüche

1. Verfahren zur selektiven quantitativen Bestimmung biologischer Spezies, **dadurch gekennzeichnet, dass** die zu bestimmende Spezies mit einer auf einem Resonanzkörper angeordneten Schicht aus für sie spezifischen Antikörpern in Kontakt gebracht wird und eine Verschiebung der Resonanzfrequenz des angeregten Resonanzkörpers infolge der Massenveränderung durch gebundene Spezies gemessen wird.

2. Sensor zur selektiven quantitativen Bestimmung biologischer Spezies, **gekennzeichnet durch**, einen Resonanzkörper (2, 13) eine auf einer Oberfläche des Resonanzkörpers angeordnete Schicht (4) mit Antikörpern für die zu bestimmende Spezies, Mittel (7,8) zur Schwingungsanregung im Resonanzkörper und Mittel (9, 12) zur Messung der Resonanzfrequenz des Resonanzkörpers.

3. Sensor nach Anspruch 2, **dadurch gekennzeichnet, dass** der Resonanzkörper eine Federzunge (2) ist.

4. Sensor nach Anspruch 3, **dadurch gekennzeichnet, dass** die Mittel zur Schwingungsanregung aus einem auf der Federzunge angebrachten Widerstand (7) zur elektrothermischen Biegung der Federzunge bestehen.

5. Sensor nach Anspruch 3, **dadurch gekennzeichnet, dass** die Mittel zur Schwingungsanregung aus an der Unterseite der Federzunge (2) und im Abstand unterhalb derselben angeordneten Elektroden (8) zur elektrostatischen Biegung der Federzunge bestehen.

6. Sensor nach Anspruch 3, **dadurch gekennzeichnet, dass** die Mittel zur Schwingungsanregung aus einer auf der Federzunge angebrachten Schicht aus piezoelektrischem Material bestehen.

7. Sensor nach Anspruch 3, **dadurch gekennzeichnet, dass** die Mittel zur Schwingungsanregung aus einem ferromagnetischen Material an der Federzunge und einer unterhalb derselben angeordneten Spule bestehen.

8. Sensor nach Anspruch 3, **dadurch gekennzeichnet, dass** die Mittel zur Messung der Resonanzfrequenz aus an der Unterseite der Federzunge (2) und im Abstand unterhalb derselben angeordneten Elektroden (9) zur elektrostatischen Biegung der Federzunge bestehen.

9. Sensor nach Anspruch 3, **dadurch gekennzeichnet, dass** die Mittel zur Messung der Resonanzfrequenz aus piezoresistive Widerstände (12) bestehen, die in die Federzunge eingebracht und als Wheatstone-Brücke verschaltet sind.

10. Sensor nach Anspruch 3, **dadurch gekennzeichnet, dass** die Mittel zur Messung der Resonanzfrequenz aus einem ferromagnetischen Material an der Federzunge und einer unterhalb derselben angeordneten Spule bestehen.

11. Sensor nach Anspruch 2, **dadurch gekennzeichnet, dass** der Resonanzkörper ein piezoelektrisches Substrat (13) ist.

12. Sensor nach Anspruch 4, **dadurch gekennzeichnet, dass** auf einer Oberfläche des Substrats kammförmige Anregungselektroden (15) und Detektorelektroden (16) und beiderseits der Elektroden Reflektoren (17) aufgebracht sind.
